Europäisches Patentamt

European Patent Office

Office européen des brevets

(19)

(11) **EP 0 781 408 B1**

(12) **EUROPÄISCHE PATENTSCHRIFT**

(45) Veröffentlichungstag und Bekanntmachung des Hinweises auf die Patenterteilung:
**22.07.1998  Patentblatt 1998/30**

(51) Int Cl.$^6$: **G01N 27/12**, G01N 33/00

(21) Anmeldenummer: **95930388.4**

(86) Internationale Anmeldenummer:
**PCT/DE95/01242**

(22) Anmeldetag: **11.09.1995**

(87) Internationale Veröffentlichungsnummer:
**WO 96/08712 (21.03.1996 Gazette 1996/13)**

(54) **GASSENSOR**

GAS SENSOR

DETECTEUR DE GAZ

(84) Benannte Vertragsstaaten:
**AT BE DE DK ES FR GB IT NL**

(30) Priorität: **14.09.1994  DE 4432729**

(43) Veröffentlichungstag der Anmeldung:
**02.07.1997  Patentblatt 1997/27**

(73) Patentinhaber: **SIEMENS AKTIENGESELLSCHAFT
80333 München (DE)**

(72) Erfinder:
• **FRANK, Joachim
D-85521 Ottobrunn (DE)**

• **FLEISCHER, Maximilian
D-85635 Höhenkirchen (DE)**
• **MEIXNER, Hans
D-85540 Haar (DE)**

(56) Entgegenhaltungen:
EP-A- 0 056 752        EP-A- 0 464 243
EP-A- 0 527 259        EP-A- 0 563 613
DE-C- 4 203 522        US-A- 5 298 783

• **PATENT ABSTRACTS OF JAPAN vol. 018 no. 596 (P-1825) ,14.November 1994 & JP,A,06 222026 (TOYOTA MOTOR CORP) 12.August 1994,**

**Beschreibung**

Die Erfindung betrifft einen Gassensor auf der Basis von Galliumoxid zur Erfassung oxidierender oder reduzierender Gase.

Als Sensorsignal herkömmlicher Galliumoxid-Gassensoren dient bisher der absolute elektrische Widerstand der gassensitiven Schicht, wie dies aus der EP 0 464 243 "Sauerstoffsensor mit halbleitendem Galliumoxid" oder der EP 0 464 244 "Sensor zur Erfassung reduzierender Gase" bekannt ist. Der elektrische Widerstand, wie auch die Gassensitivität von $Ga_2O_3$ sind stark temperaturabhängig. Um einen gassensitiven Effekt zu erzielen, müssen die $Ga_2O_3$ Gassensoren in einem gastypischen Temperaturbereich betrieben werden. In diesen mußten sie bisher sehr genau thermostatisiert werden, damit eine Temperaturschwankung nicht die gleiche Widerstandsänderung hervorruft, wie sie durch eine Änderung der Konzentration des zu detektierenden Gases verursacht werden würde. Um eine hinreichend konstante Betriebstemperatur zu gewährleisten, ist ein hoher geratetechnischer Aufwand notwendig. Die Empfindlichkeit des Gesamtsystems wird stark von der bleibenden Regelabweichung begrenzt. Bei starken und häufigen Temperaturschwankungen ist eine Signalauswertung nur mehr bedingt möglich, da die Regelung nur verzögert reagiert.

Wird ein Teil der Sensoranordnung mit einer gasundurchlässigen Schicht abgedeckt, um diesen dadurch passivierten Teil zur Temperaturkompensation zu verwenden, wie dies in den Weiterbildungen der EP 0 464 243 oder der EP 0 464 244 angegeben ist, ergibt sich folgendes Problem. Die anfangs gasundurchlässige Schicht, die auf einen gassensitiven Teil zu dessen Abdeckung aufgebracht ist, wird mit der Zeit rissig und somit gasdurchlässig. Der als lediglich temperaturabhängig vorgesehene Teil der Sensoranordnung wird zusätzlich durch das durch die Risse in der Abdeckschicht dringende Gas gassensitiv, was die Messung erheblich verfälscht.

Eine weitere Möglichkeit einen Teil der Sensoranordnung zur Temperaturkompensation zu nutzen, besteht darin, diesen mit Metallatomen, wie beispielsweise Gold derat hoch zu dotieren, daß dieser seine Gassensitivität verliert. Beschrieben ist dies in der DE 42 10 397 oder der DE 42 10 398. Nachteilig daran ist, daß der dotierte Teil der Sensoranordnung nicht stabil ist.

Die Aufgabe der Erfindung ist es, einen Gassensor anzugeben, der zwei Ausgangssignale zur Verfügung stellt, wobei das eine eine Gas- und eine Temperaturabhängigkeit und das andere lediglich die Temperaturabhängigkeit aufweist.

Die Aufgabe wird durch einen Gassensor gemäß dem Patentanspruch 1 gelöst.

Eine geeignete Verknüpfung der beiden Ausgangssignale liefert vorteilhafter Weise ein von Temperaturschwankungen unabhängiges Signal.

Vorteilhafte Weiterbildungen ergeben sich aus den Unteransprüchen.

Die Erfindung wird im folgenden anhand der Figuren näher erläutert.

Figur 1 zeigt schematisch den Aufbau des gassensitiven Teils und des nicht gassensitiven Teils des Gassensors.

Figur 2 zeigt den prinzipiellen Aufbau des Gassensors in der Draufsicht.

Figur 3 zeigt den Widerstand in Abhängigkeit von der Temperatur zum einen für den gassensitiven Sensorteil und zum anderen für den nicht gassensitiven Sensorteil.

Figur 4 zeigt die Abhängigkeit des elektrischen Widerstands vom Sauerstoffpartialdruck, wiederum sowohl für den gassensitiven Teil und den nicht gassensitiven Teil des Sensors.

Figur 5 zeigt die unterschiedlichen Sensitivitäten der beiden Sensormaterialien in Abhängigkeit von der Temperatur für $CH_4$.

Figur 6 zeigt die unterschiedlichen Sensitivitäten der beiden Sensormaterialien in Abhängigkeit von der Temperatur für $H_2$.

Figur 7 zeigt die unterschiedlichen Sensitivitäten der beiden Sensormaterialien in Abhängigkeit von der Temperatur für CO.

Figur 8 zeigt eine mögliche Auswerteelektronik.

Figur 9 zeigt eine weitere mögliche Auswerteelektronik.

Figur 10 zeigt eine dritte mögliche Auswerteelektronik.

Figur 11 zeigt eine vierte mögliche Auswerteelektronik.

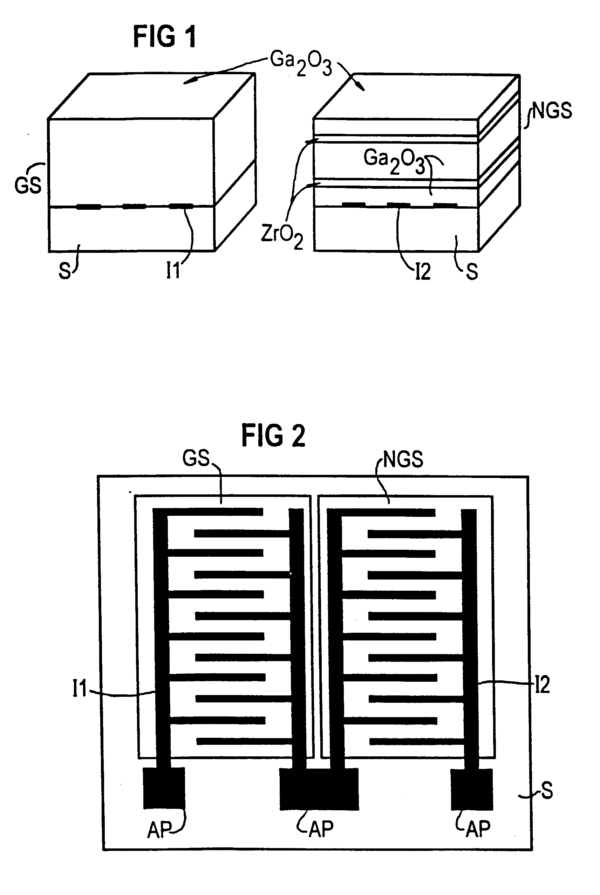

Durch Verwendung des in Figur 1 rechts gezeigten Referenzwiderstandes, dem nicht gassensitiven Teil des Gassensors NGS und dem elektrischen Widerstand des gassensitiven Sensorteils GS, vergleiche Figur 1 links, kann das Verhältnis oder die Differenz zwischen den beiden Widerständen verglichen werden, um ein Signal zu erzeugen, das nur mehr von der Gasart und nicht mehr von Temperaturschwankungen abhängig ist. Beide Teile NGS und GS des Gassensors besitzen die gleiche Abhängigkeit des elektrischen Widerstands von der Temperatur.

Die gassensitive Anordnung, vergleiche Figur 1 links, wird durch reaktives Sputtern hergestellt, wobei als gassensitives Material von einer einem n-Typ-Halbleiter, zum Beispiel $Ga_2O_3$ - Keramik ausgegangen wird. Dem Sputtergas Argon werden hierzu ca. 25% Sauerstoff beigegeben. Die nicht gassensitive Anordnung NGS wird ebenso hergestellt mit dem Unterschied, daß in diese Anordnung eine oder mehrere Zwischenlagen eines p-Typ-Halbleiters, zum Beispiel $ZrO_2$ als nicht gassensitives Material eingebracht werden. Hierfür wird der $Ga_2O_3$ - Sputterprozeß unterbrochen. Das

EP 0 781 408 B1

Material der Zwischenschicht $ZrO_2$ wird ebenfalls reaktiv unter Zugabe von Sauerstoff gesputtert. Hierauf folgt wieder eine Schicht $Ga_2O_3$ usw.. Die Gesamtschichtdicken für $Ga_2O_3$ und $ZrO_2$ sind so zu wählen, daß ca. 5 bis 20% der Gesamtschichtdicke der Anordnung aus $ZrO_2$ besteht.

Durch Variationen der Schichtdicken können gegebenenfalls die Widerstände einander angeglichen werden.

Die Gesamtschichtdicke der jeweiligen Anordnung liegt zwischen 1 - 10µm, wobei typischerweise 1 - 4 Schichten verwendet werden.

$ZrO_2$ ist ein gasdurchläßiges Material. Die Gasdurchläßigkeit ist jedoch für die Funktionstüchigkeit des Sensors kein entscheidendes Kriterium. Vielmehr ist darauf zu achten, daß im nicht gassensitiven Teil NGS des Sensors sich Schichten aus p- und n-Typ-Halbleitern befinden.

Das Herstellverfahren läuft wie folgt ab. Zuerst wird auf dem Substrat S die nicht gassensitive Anordnung NGS erzeugt und dabei der Bereich abgedeckt, wo später die gassensitive Anordnung GS erzeugt werden soll. Anschließend wird die gassensitive Anordnung GS hergestellt und dabei der Bereich abgedeckt, in dem bereits der nichtgassensitive Gassensorteil NGS erzeugt wurde. Durch diese Reihenfolge wird sichergestellt, daß das $ZrO_2$ auf keinen Fall die gassensitive Anordnung GS verunreinigt.

Da sich, wie in Figur 2 gezeigt, der Referenzwiderstand auf dem gleichen Substrat S wie der gassensitive Teil GS und in dessen unmittelbarer Nähe befindet, erfahren beide die gleichen Temperaturschwankungen. Bei der durch den Aufbau des Referenzteils (nicht gassensitiver Teil) gegebenen gleichen Temperaturabhängigkeit ändern der gassensitive Sensorteil und der Referenzteil bei Temperaturschwankungen ihren elektrischen Widerstand gleichermaßen. Das Verhältnis oder die Differenz von Sensor- und Referenzwiderstand wird durch eine Temperaturschwankung nicht beeinflußt. Die Temperatur muß somit nur in dem Bereich gehalten werden, indem der gewünschte Gaseffekt auftritt. Das heißt, wenn der Gassensor für die Detektion reduzierender Gase verwendet werden soll, ist die Betriebstemperatur zwischen 600° und 850°C zu halten. Soll der Gassensor jedoch für die Detektion von Sauerstoff verwendet werden, ist die Betriebstemperatur bei ca. 950°C einzustellen.

Die mittleren beiden Anschlüsse AP (Anschlußpads) der Interdigital-Elektrodenstrukturen I1, I2 sind vorteilhafterweise miteinander verbunden. Dies ist jedoch nicht zwingend erforderlich.

Der erfindungsgemäße Gassensor weist folgende Vorteile auf:

a) Die Anforderung an die Temperaturregelung werden geringer gegenüber dem eingangs genannten Stand der Technik. Die Temperaturschwankungen können sich über den ganzen Temperaturbereich erstrecken in dem der gewünschte Gaseffekt in entsprechender Größe auftritt.

b) Eine Widerstandsdrift des gassensitiven Teils aufgrund von Alterung wirkt sich nicht mehr auf das Sensorsignal aus, da der Referenzwiderstand den gleichen Alterungseffekten unterliegt.

c) Durch Verwendung eines Widerstandsverhältnisses oder einer Widerstandsdifferenz umgeht man das Problem, hohe Sensorwiderstände auswerten zu müssen. Die Anforderungen an die Auswerteelektronik sinken dadurch.

d) Schwankungen und Unsicherheiten im Herstellungsprozeß des Gassensors wirken sich bei dem beschriebenen Verfahren weniger auf das Sensorsignal aus. Der nichtgassensitive Teil NGS, der Referenzwiderstand, ist auf dem gleichen Substrat S aufgebracht wie der gassensitive Teil, der eigentliche Gassensor. Bis auf das Abscheiden der gasoder nichtgassensitiven Anordnung durch Sputtern durchlaufen beide die gleichen Prozesse und somit weitestgehendst die gleichen technologischen Schwankungen.

e) Wechselwirkungen der gassensitiven Anordnung mit dem Substrat S wirken sich nicht mehr so stark auf das Sensorsignal aus. Bisher verwendete Substrate, wie BeO oder $Al_2O_3$ führen aufgrund von Interdiffusion zu einer mehr oder weniger starken Widerstandszunahme bei hohen Betriebstemperaturen.

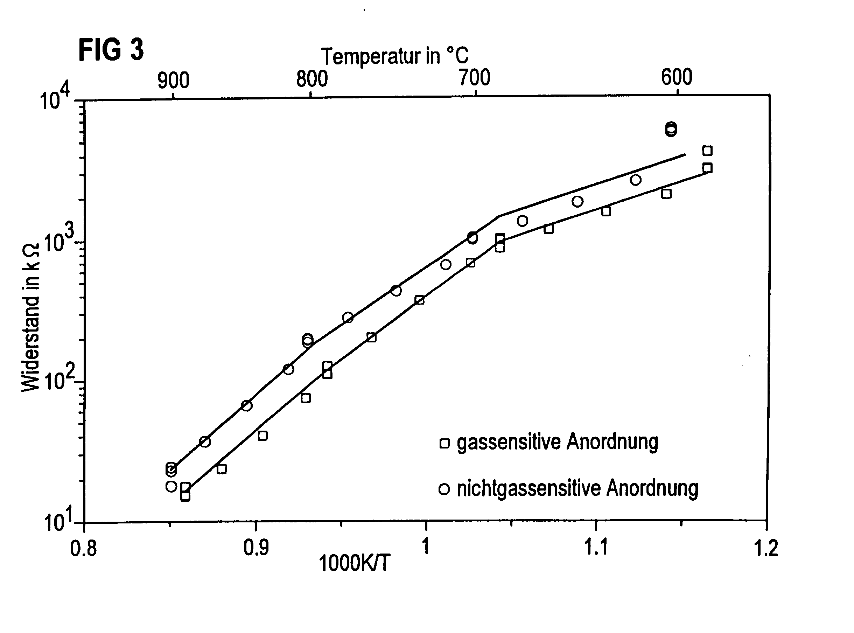

Mit Hilfe der Diagramme aus den Figur 3 bis 7 lassen sich die verbesserten Eigenschaften des Gassensors verdeutlichen.

Aus Figur 3 ist ersichtlich, daß sich der Widerstand der gassensitiven Anordnung GS gegenüber dem der verwendeten nicht gassensitiven Anordnung NGS in einem weiten Temperaturbereich auf die gleiche Art und Weise von der Temperatur abhängend ändert.

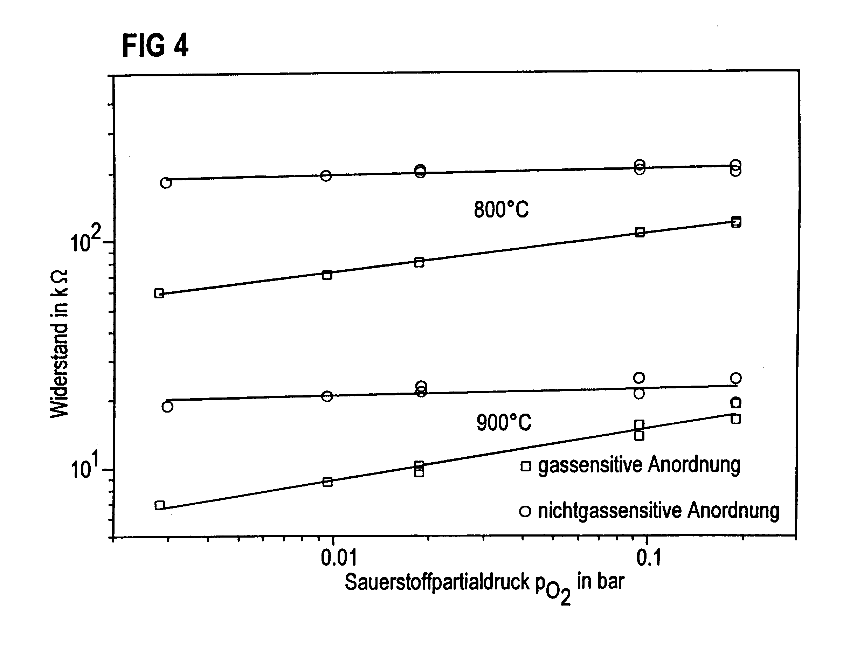

In Figur 4 ist deutlich zu erkennen, daß der Widerstand der verwendeten nicht gassensitiven Anordnung NGS sich bei einer Änderung des Partialdrucks des zu detektierenden Sauerstoffs nicht nennenswert ändert. Hingegen weist der Widerstand der gassensitiven Anordnung GS, wie gewünscht, eine Abhängigkeit vom Sauerstoffpartialdruck auf.

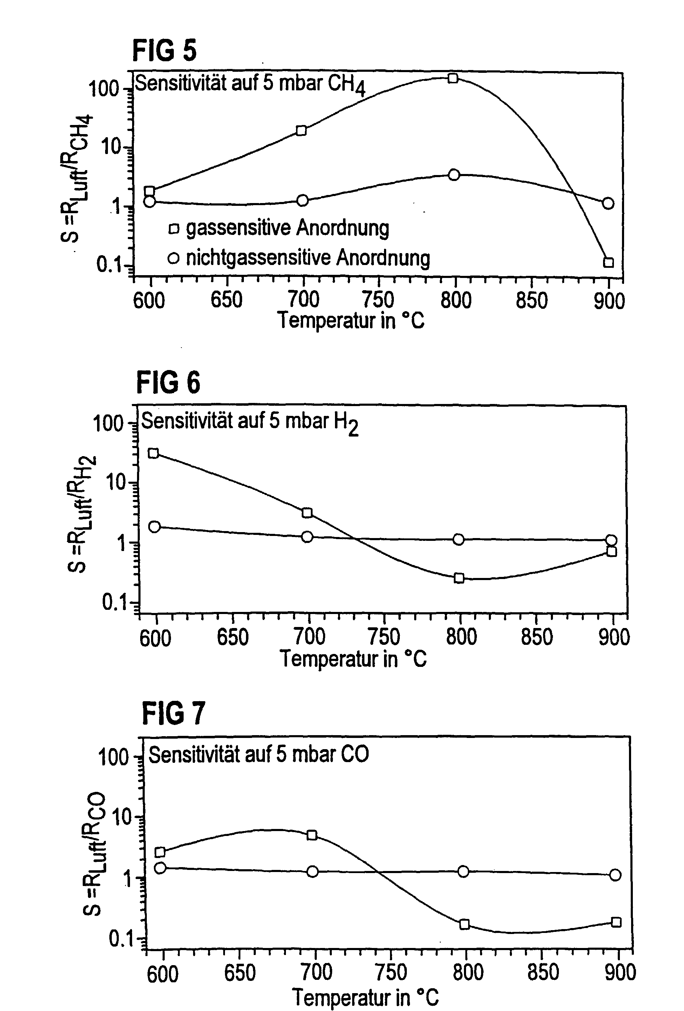

In den Figuren 5, 6 und 7 ist die Sensitivität für verschiedene reduzierende Gase in Abhängigkeit von der Betriebstemperatur sowohl für die gassensitive Anordnung GS als auch die nicht gassensitive Anordnung NGS aufgetragen. Die besten Ergebnisse bei dem aufgezeichneten Temperaturschwankungsbereich von 600 bis 900°C ergeben sich für

$H_2$ und CO.

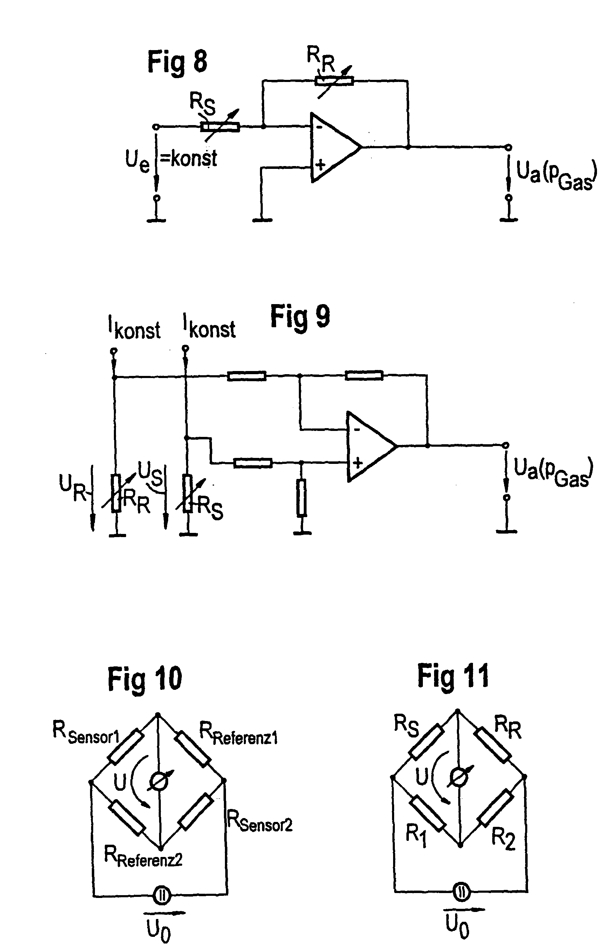

Eine mögliche Auswerteelektronik für die vom Gassensor gelieferten Ausgangssignale stellt Figur 8 dar. Hierbei wird das Widerstandsverhältnis des Referenzteils NGS zum gassensitiven Teil GS gemessen. Die Empfindlichkeit A ergibt sich zu:

$$A = \frac{U_a}{U_e} = - \frac{R_R(T)}{R_S(T,p_{Gas})}$$

$U_a =$ Ausgangsspannung
$U_e =$ Eingangsspannung (= konstant)
$R_R(T) =$ temperaturabhängiger Widerstand des Referenzteils NGS
$R_S(T, p_{GAS}) =$ temperatur- und gasabhängiger Widerstand des gassensitiven Teils GS

In Figur 9 ist eine weitere Variante der Auswerteelektronik gezeigt. Die meßbare Widerstandsdifferenz zwischen dem Referenzteil NGS und dem gassensitiven Teil GS ergibt sich zu:

$$U_a = U_R - U_S$$

$$U_a = I_{const}[R_R(T) - R_S(T,p_{Gas})]$$

$$U_a = I_{const}[R_{R0} - R_{S0} - \Delta R_{Gas}]$$

Die Widerstände $R_{R0}$ und $R_{S0}$ sind konstante Werte. Der Widerstand $\Delta R_{Gas}$ hingegen ist vom Gas abhängig.

Für die in Figur 10 gezeigte dritte Ausführungsform der Auswerteelektronik, bei der zwei Gassensoren zu einer Brücke verschaltet sind, ergibt sich die gemessene Spannung U zu:

$$U = \frac{R_S(T, p_{Gas}) - R_R(T)}{R_S(T, p_{Gas})} \cdot U_0$$

wobei:

$R_S(T, p_{Gas}) = R_{Sensor1}(T, p_{Gas}) = R_{Sensor2}(T, p_{Gas})$
$R_R(T) = R_{Referenz1}(T) = R_{Referenz2}(T)$
$U_0 = $ Betriebsspannung

Für die in Figur 11 gezeigte vierte Ausführungsform der Auswerteelektronik, bei der ein Gassensor und zwei Festwiderstände $R_1$ und $R_2$ zu einer Brücke verschaltet sind, ergibt sich die gemessene Spannung U zu:

$$U = \left( \frac{R_S(T, p_{Gas})}{R_S(T, p_{Gas}) - R_R(T)} - \frac{R_1}{R_1 + R_2} \right) \cdot U_0$$

Die Schichten werden auf zwei benachbarten Interdigital-Elektrodenstrukturen I1, I2 aufgebracht.

Eine anschließende Temperung bei 900 bis 1050°C stellt definierte Bedingungen für spätere Messungen bei 900°C her. Die Temperung führt nicht zur einer Ausdiffusion der Zwischenschichten.

Der Gassensor ist bei Temperaturen von 600 bis 850°C für die Detektion reduzierender Gase geeignet. Bei 950°C dient er als Sauerstoffsensor.

Als Substart S eignet sich beispielsweise Quarzglas.

Der Gassensor ist unter anderem einsetzbar für Methan- oder Kohlenmonoxidwarngeräte, für Kleinfeuerungsanlagen oder für die Verbrennungsregelung.

**Patentansprüche**

1. Gassensor,

 - bei dem auf einem Substrat (S) zwei in unmittelbarer Nähe zueinander befindliche Elektrodenstrukturen (I1, I2) angeordnet sind, und
 - bei dem die eine Elektrodenstruktur (I1) von einer einen n-Typ-Halbleiter aufweisenden gassensitiven Anordnung (GS), deren Widerstand sowohl gas- als auch temperaturabhängig ist, umgeben ist, und
 - bei dem die andere Elektrodenstruktur (I2) von einer einen n-Typ-Halbleiter und einen p-Typ-Halbleiter aufweisenden nicht gassensitiven Anordnung (NGS) umgeben ist, deren Widerstand nur temperaturabhängig ist.

2. Gassensor nach Anspruch 1,

 - bei dem der n-Typ-Halbleiter der gassensitiven Anordnung (GS) $Ga_2O_3$ ist, und
 - bei dem der n-Typ-Halbleiter der nicht gassensitiven Anordnung (NGS) $Ga_2O_3$ und der p-Typ-Halbleiter der nicht gas-sensitiven Anordnung (NGS) $ZrO_2$ ist.

3. Gassensor nach Anspruch 1 oder 2,
 bei dem die nicht gassensitive Anordnung (NGS) wenigstens eine Schicht des n-Typ-Halbleiters und wenigstens eine Schicht des p-Typ-Halbleiters aufweist.

4. Gassensor nach Anspruch 3,
 bei dem die Gesamtschichtdicke des p-Typ-Halbleiters im Bereich von 0,05 bis 0,2 der Gesamtschichtdicke der nicht gassensitiven Anordnung (NGS)liegt.

5. Gassensor nach Anspruch 3 oder 4,
 bei dem die Schichten bei 900 - 1050°C getempert sind.

6. Gassensor nach einem der Ansprüche 1 - 5,
 bei dem die Elektrodenstruktur (I1, I2) eine Interdigitalstruktur aufweist.

7. Verwendung des Gassensors nach einem der Ansprüche 1 - 6 bei 600 - 850°C zur Detektion von reduzierenden Gasen.

8. Verwendung des Gassensors nach einem der Ansprüche 1 - 6 bei 950°C zur Detektion von Sauerstoff.


**Claims**

1. Gas sensor,

 - in which two electrode structures (I1, I2), located directly next to each other, are arranged on a substrate (S), and
 - in which one (I1) of the electrode structures is surrounded by a gas-sensitive arrangement (GS) which comprises an n-type semiconductor and whose resistance is both gas-dependent and temperature-dependent, and
 - in which the other (I2) of the electrode structures is surrounded by a non-gas-sensitive arrangement (NGS) which comprises an n-type semiconductor and a p-type semiconductor and whose resistance is only temperature-dependent.

2. Gas sensor according to Claim 1,

 - in which the n-type semiconductor of the gas-sensitive arrangement (GS) is $Ga_2O_3$, and
 - in which the n-type semiconductor of the non-gas-sensitive arrangement (NGS) is $Ga_2O_3$ and the p-type semiconductor of the non-gas-sensitive arrangement (NGS) is $ZrO_2$.

3. Gas sensor according to Claim 1 or 2, in which the non-gas-sensitive arrangement (NGS) comprises at least one layer of the n-type semiconductor and at least one layer of p-type semiconductor.

4. Gas sensor according to Claim 3, in which the total layer thickness of the p-type semiconductor is in the range from 0.05 to 0.2 of the total layer thickness of the non-gas-sensitive arrangement (NGS).

5. Gas sensor according to Claim 3 or 4, in which the layers are heat-treated at 900-1050°C.

6. Gas sensor according to one of Claims 1-5, in which the electrode structure (I1, I2) comprises an interdigital structure.

7. Use of the gas sensor according to one of Claims 1-6 at 600-850°C for the detection of reducing gases.

8. Use of the gas sensor according to one of Claims 1-6 at 950°C for the detection of oxygen.

**Revendications**

1. Détecteur de gaz

   - dans lequel deux structures d'électrodes (I1, I2) adjacentes sont disposées sur un substrat (S), et
   - dans lequel une structure d'électrodes (I1) est entourée par un montage sensible aux gaz (SG) doté d'un semiconducteur de type N dont la résistance dépend aussi bien des gaz que de la température et
   - dans lequel l'autre structure d'électrodes (I2) est entourée par un montage insensible aux gaz (IG) doté d'un semiconducteur de type N et d'un semiconducteur de type P dont la résistance dépend uniquement de la température.

2. Détecteur de gaz selon la revendication 1

   - dans lequel le semiconducteur de type N du montage sensible aux gaz (SG) est constitué de $Ga_2O_3$ et
   - dans lequel le semiconducteur de type N du montage insensible aux gaz (IG) est constitué de $Ga_2O_3$ et le semiconducteur de type P du montage insensible aux gaz (IG) de $ZrO_2$.

3. Détecteur de gaz selon la revendication 1 ou 2
   dans lequel le montage insensible aux gaz (IG) présente au moins une couche de semiconducteur de type N et au moins une couche de semiconducteur de type P.

4. Détecteur de gaz selon la revendication 3
   dans lequel l'épaisseur totale de la couche de conducteur de type P se situe dans la plage de 0,05 à 0,2 de l'épaisseur totale des couches du montage insensible aux gaz (IG).

5. Détecteur de gaz selon la revendication 3 ou 4
   dans lequel les couches subissent un recuit à une température de 900 - 1050°C.

6. Détecteur de gaz selon l'une des revendications 1 à 5
   dans lequel la structure des électrodes (11, 12) comprend une structure interdigitale.

7. Utilisation du détecteur de gaz selon l'une des revendications 1 à 6 à une température de 600 - 850 °C en vue de la détection de gaz réducteurs.

8. Utilisation du détecteur de gaz selon l'une des revendications 1 à 6 à une température de 950 °C en vue de la détection de l'oxygène.

## FIG 1

$Ga_2O_3$
NGS
GS
$Ga_2O_3$
$ZrO_2$
S
I1
I2
S

## FIG 2

GS
NGS
I1
I2
S
AP
AP
AP

FIG 3

Temperatur in °C

Widerstand in k Ω

1000K/T

□ gassensitive Anordnung

o nichtgassensitive Anordnung

FIG 4

## FIG 5

S = $R_{Luft}/R_{CH_4}$

Sensitivität auf 5 mbar CH$_4$

□ gassensitive Anordnung
○ nichtgassensitive Anordnung

Temperatur in °C

## FIG 6

S = $R_{Luft}/R_{H_2}$

Sensitivität auf 5 mbar H$_2$

Temperatur in °C

## FIG 7

S = $R_{Luft}/R_{CO}$

Sensitivität auf 5 mbar CO

Temperatur in °C

Fig 8

Fig 9

Fig 10

Fig 11

11